(19) Europäisches Patentamt
European Patent Office
Office européen des brevets

(11) Publication number: 0 184 603 A1

(12) EUROPEAN PATENT APPLICATION

(21) Application number: 84850382.7

(22) Date of filing: 07.12.84

(51) Int. Cl.[4]: D 21 C 9/00, D 21 D 3/00

(43) Date of publication of application: 18.06.86 Bulletin 86/25

(84) Designated Contracting States: DE FR GB IT

(71) Applicant: KORSNÄS-MARMA AB, Box 592, S-801 11 Gävle (SE)

(72) Inventor: Schönfeldt, Nikolaus, Häradsgatan 30B, S-431 42 Mölndal (SE)
Inventor: Parck, Curt, Kyrkängsvägen 16, S-802 40 Gävle (SE)
Inventor: Hakansson, Sven, Kornsnäsvägen 38, S-802 38 Gävle (SE)

(74) Representative: Larfeldt, Helene et al, Axel Ehrners Patentbyrä AB P.O. Box 5342, S-102 46 Stockholm (SE)

# (54) Process for preparing a fluff pulp.

(57) A process in preparing a high absorption fluff pulp which can be shredded at a low power consumption to fluff, wherein to the pulp suspension before or on the wire is added ampholytic tensides and/or tensides which are cationic at low pH values and nonionic at high pH values, the pH value being kept sufficiently low for the tensides to be in cationic form, whereupon the pH value is increased to such an extent that the tensides are transferred into the anionic and nonionic form respectively, which pH increase takes place just before the pulp web leaves the wire or before any of the presses or between the last press and the drying section or in the drying section or in connection with the feeding of the pulp into the fluffing machine.

EP 0 184 603 A1

PROCESS FOR PREPARING A FLUFF PULP.

This invention refers to a process in preparing a high absorption fluff pulp from a pulp suspension on a pulp-drying machine, wherein, to improve fiberizing, to the pulp suspension before or on the wire is added a debonding agent and optionally, to improve the absorption, an absorption promoting agent.

It is known to use fluff produced by dry fiberizing of fluff pulp, such as cellulose pulp in the form of web rolls or pulp bales, for absorbent media in sanitary products such as diapers and sanitary napkins.

Fluff pulp is conventionally prepared on a pulp drying machine having a wet-end comprising a wire and a press section and a drying section. In order to reduce the tearing power and to facilitate the fiberizing, the pulp before or on the wire can be provided with certain additives which reduce the bonding and the friction between the fibres in the pulp. In adding debonding agents it should, however, be observed that they can have an injurious effect on the remaining characteristics of the fluff which are desirable in technical use, for instance fluid absorption time, high brightness, high bulk and good elasticity characteristics.

From US Patent 3,395,708 it is known to add cationic tensides to cellulose pulp in order to facilitate the fiberizing. The cationic tensides generally are substantive and consequently adhere well to the fibres. The addition of cationic tensides, for instance quaternary ammonium compounds, however, has a hydrophobic influence on the fibres and is accordingly detrimental to the fluid absorption ability of the fluff pulp and often to its brightness too. In order to avoid these disadvantages cationic tensides have been combined with absorption promoting agents, for instance nonionic tensides.

Cationic tensides remain cationic both in alkaline and acid environment. The tendency to make the fibres hydrophobic could then only be compensated for by, as stated above, mixing with an absorption promoting agent, such as a nonionic tenside.

Said disadvantages can, however, be overcome completely or to a large extent in accordance with the invention by the use of such additives to the pulp which change characteristics when the environment from being acid becomes neutral or alkaline. According to the present invention a process in preparing a high absorption fluff pulp is characterized in that the debonding agent is an ampholytic tenside and/or a tenside, which depending on the pH value is cationic or nonionic, said tenside(s) is added in an amount of 0.01-2% by weight based on the dry weight of the pulp suspension, the pH value of the pulp suspension at the addition is kept sufficiently low for the tenside(s) to be in cationic form, one or more absorption promoting agents is added, if required, and the pH value of the pulp after dewatering on the wire is increased to such an extent that the debonding tenside(s) is tranferred into the anionic and nonionic form respectively.

Suitable absorption promoting agents are anionic tensides and nonionic tensides. As examples can be mentioned straight or branched aliphatic alcohols having at least 8 carbon atoms in the alkyl chain, carboxylic amides with at least 12 carbon atoms in the alkyl chain and ethanol amides of straight or branched aliphatic hydrocarbons with at least 12 carbon atoms in the hydrocarbon chain.

The debonding tensides are added to the pulp before or on the wire in an acid environment, that is at pH values below 7. By increasing the pH value of the pulp the additives are transferred from cationic form into the anionic and nonionic form respectively (depending on the nature of the

additives). By this the fluid absorption capacity of the final product is increased. This increase of the pH value must be performed not to loose the intended effect of the additives on the fiberizing of the pulp and should therefore take place after a substantial reduction of the water content of the pulp.

According to the invention the increase of the pH value can take place as follows:

An alkaline liquid is supplied to the web

a. just before the pulp web leaves the wire
b. immediately before one or more of the presses
c. between the last press and the drying section
d. in the drying section, the addition being made so quickly that the web will be moistened through before the liquid evaporates to any considerable extent
e. in connection with the feeding of the pulp to the fluffing machine (shredder).

By this change of the characteristics of the tenside, after having served the purpose to prevent the forming of fibre bonds, its tendency to make the fibres hydrophobic is compensated for. The liquid absorption capacity of the fibres is increased by this. If required this effect could be further strengthened by the addition of a tenside mixture to the pulp before or on the wire, which mixture comprises an ampholytic and/or cationic/nonionic tenside to facilitate the fiberizing and a non-cationic tenside to increase the liquid absorption capacity, for instance a nonionic or an anionic tenside.

A. Examples of ampholytic tensides which are cationic in acid and anionic in alkaline media are:

a) aminocarboxylic acids or aminosulphonic acids of the general formula

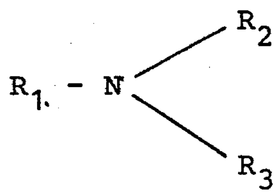

wherein $R_1$ is a straight or branched chain, saturated or unsaturated alkyl group with 8-24 carbon atoms, $R_2$ is hydrogen, a short alkyl group with 1-6 carbon atoms or a chain containing 1-40 oxyethylene groups, and $R_3$ is a residue containing a group -COOH or $-SO_3H$. As an example can be mentioned.

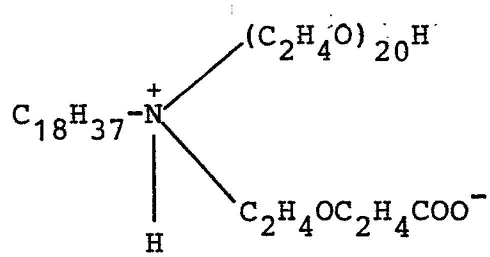

In addition should be mentioned betaines. These compounds are internal ammonium salts and can contain one or more amino groups. As an example could be mentioned betaines of the general formula

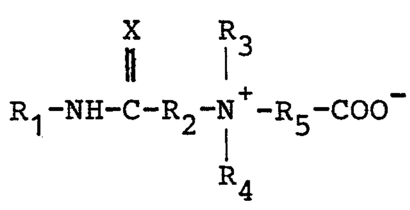

wherein $R_1$ is a straight or branched, saturated or unsaturated alkyl group with 8-24 carbon atoms, $R_2$ and $R_5$ are alkylene groups with 1-2 carbon atoms, $R_4$ is a group $-C_nH_{2n+1}$ or $-C_nH_{2n}OH$ wherein n is an integer between 1 and 10, or $-(C_2H_4O)_xH$ wherein x is an integer between 2 and 100, $R_3$ is $R_4$ or a group

$R_1$-NH-C-$R_2$,-$R_5$-COOH or -$R_2$-COOMe, wherein $R_1$, $R_2$, $R_4$ and $R_5$ are as defined above, Me is a monovalent, salt forming radical and X is O or S. Among betaines the following, which are commercial products, could be mentioned: stearyl betaine, soyaammonium sulphobetaine, palmityl ammonium carboxylic betaine and tallow amido ammonium sulphobetaine.

b) Imidazoline derivates.

These products are prepared from imidazoline compounds of the general fomula:

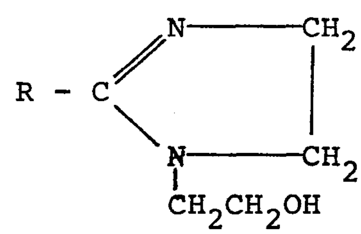

which are carboxymethylated under the addition of one or more groups $-CH_2COO$ or sulphonated or sulphated. R is a straight or branched, saturated or unsaturated alkyl group with 8-24 carbon atoms. As an example could be mentioned a commercial product, Miranol C2M, which in anhydrous acid form has the following formula.

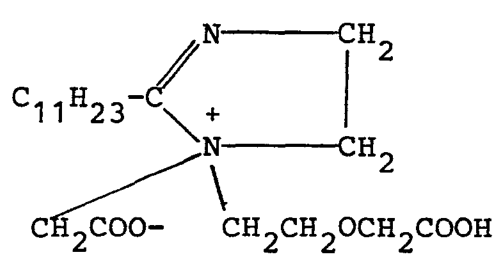

B. Examples of tensides which are cationic in acid and nonionic in alkaline or neutral media are alkylene oxide derivates of

a) monoamines comprising

1. N-primary alkyl amines with 10-22 carbon atoms in a straight or branched, saturated or unsaturated alkyl group, for instance tallow fatty amine or stearyl amine.

2. tertiary alkyl primary amines, for instance $t\text{-}C_{12-14}NH_2$ or $t\text{-}C_{18-22}NH_2$

3. resin amine for instance dehydroabiethylamine.

b) polyamines comprising

1. N-alkyl-alkylene diamines with 12-24 carbon atoms in a the alkyle group which can be straight or branched, saturated or unsaturated and with 1-12 carbon atoms in the alkylene group, for instance tallow fatty amine propylene diamine and

2. alkyl polyalkylene polyamines of the general formula $R\text{-}NH\text{-}(C_nH_{2n}\text{-}NH)_x\text{-}C_nH_{2n}\text{-}NH_2$, wherein R is an alkyl group which can be straight or branched, saturated or unsaturated and contain 12-24 carbon atoms, n is an integer between 1 and 12 and x is an integer between 1 and 8.

Alkylene oxide derivatives of said amines are obtained by addition of ethylene oxide or ethylene oxide and a higher alkylene oxide containing 3-6 carbon atoms, wherein the higher alkylene oxide can be added before, together with or after the ethylene oxide.

EXAMPLE 1

Test with an ampholytic debonding agent consisting of a complex tallow ammonium carboxylate.

The debonding agent was added to a 2.5% slurry of sulphate pulp fibres at pH 4.5. After stirring of the suspension for 10 minutes it was diluted to a fibre concentration of 0.5% and formed into sheets (dry basis weight 750 $g/m^2$) in a laboratory sheet mould.

One part of these test sheets (sample B) was pressed without a pH change to a dry content of 40%. The remaining sheets (sample C) were pressed to a dry content of 30%, whereupon the pH of the sheets was increased to 10.5 by spraying with, based on 1 g dry pulp, 10 g alkaline water, the sheets being arranged on a suction filter drawing off the surplus water. Thereafter these sheets were pressed to a dry content of 40%.

As a comparison sheets (sample A) were prepared of sulphate pulp not containing a debonding agent.

All test sheets were dried to a dry content of 93% after the pressing and were conditioned at 23°C and a relative humidity of 50%. They were then shredded to fluff on a spiked roller. The energy that was utilized for the shredding, the tearing power, was read. Fluff cushions with a diameter of 50 mm and a weight of 2 g were formed. They were pressed to a hight of 15 mm which was kept constant. The time required for water to be absorbed from the bottom of the cushion to its top, the absorption time, was measured. The results are given in Table 1.

TABLE 1

| Sample | Debonding agent, active substance/ fibres g/100 g fibre | pH at the sheet forming | pH at the final pressing | Tearing power kJ/kg | Absorption time s |
|---|---|---|---|---|---|
| A | 0 | 4.5 | 4.5 | 440 | 2.5 |
| B | 0.6 | 4.5 | 4.5 | 330 | 3.8 |
| C | 0.6 | 4.5 | 10.5 | 335 | 2.8 |

The effect of the debonding agent appears in that the tearing power has decreased from 400 kJ/kg (sample A) to about 330 kJ/kg (sample B) and the effect of the pH increase in that the absorption time has been reduced from 3.8 s (sample B) to 2.8 s (sample C), that is approximately the same value as for the untreated pulp (sample A).

EXAMPLE 2

Test with a debonding agent, which when pH is increased is transferred from cationic to nonionic form, consisting of ethoxylated tallow fatty amine having two oxyethylene groups.

The debonding agent was added to a 2.5% slurry of sulphate pulp fibres at pH 5.5. After stirring the suspension for 10 minutes it was diluted to a 0.5% fibre concentration and formed into two sheets (dry basis weight 750 g/m$^2$).

One part of the sheets (sample D) was pressed without a change of pH to a dry content of 40%.

The remaining sheets (sample E) were pressed to a dry content of 30% and then the pH of the sheets was increased to 1.0 by spraying with, based on 1 g dry pulp, 10 g alkaline water, the sheets being arranged on a suction filter to draw off surplus water. These sheets were then pressed to a dry content of 40%.

A third fluff pulp sample (sample F) was prepared by adding the debonding agent at pH 11.0 to a 2.5% slurry of sulphate pulp fibres and stirring thereof for 10 minutes, dilution and sheet forming (dry basis weight 750 $g/m^2$) and pressing to a dry content of 40% without any change of pH.

All test sheets were dried to a dry content 93% after the pressing and conditioned at 23°C and a relative humidity of 50%.

Fluff was made from the pulps and tested as in example 1. The results from the tests are stated in table 2.

The samples D and F show that pH must be low in the 2.5% fibre slurry for a debonding effect to appear, that is the agent has to be in cationic form.

TABLE 2

| Sample | Debonding agent, active substance/ fibres g/100 fibre | pH at the sheet forming | pH at the final pressing | Tearing power KJ/kg | Absorption time s |
|---|---|---|---|---|---|
| D | 0.35 | 5.5 | 5.5 | 285 | 10.5 |
| E | 0.35 | 5.5 | 11.0 | 296 | 7.4 |
| F | 0.35 | 11.0 | 11.0 | 423 | 4.1 |

Sample E shows that the increase of pH can be performed in a pressed pulp without the debonding effect to be lost. At the same time the absorption time is considerably reduced.

EXAMPLE 3

Test with a mixture of a debonding agent and a wetting agent, the debonding agent being ethoxylated tallow fatty amine containing two oxyethylene groups and having the ability to be transferred from cationic to nonionic form when pH is increased, and the wetting agent being nonionic, a $C_{12}$-$C_{14}$ alcohol adduct.

To a 2.5% slurry of sulphate pulp fibres was at pH 5.5 added the debonding agent and the wetting agent. After stirring the suspension for 10 minutes it was diluted to a 0.5% fibre concentration and formed into sheets (dry basis weight 750 g/$m^2$).

One part of the sheets (sample G) was pressed to a dry content of 40% without changing pH.

The remaining sheets (sample H) were pressed to a dry content of 30% and then the pH of the sheets was increased to 11.0 by spraying with, based on 1 g dry pulp, 10 g alkaline water, the sheet being arranged on a suction filter to draw off surplus water. These sheets were then pressed to a dry content of 40%.

All test sheets were dried to a dry content of 93% after pressing and were conditioned at 23°C and a relative humidity of 50%.

Fluff was made from the pulps and tested as in Example 1. The results are stated in Table 3. As a comparison sample D from Example 2, Table 2, has been inserted.

TABLE 3

| Sample | Debonding agent g/100 g fibre | Wetting agent g/100 g fibre | pH at the sheet forming | pH at the final pressing | Tearing power kJ/kg | Absorption time s |
|---|---|---|---|---|---|---|
| D | 0.35 | 0.0 | 5.5 | 5.5 | 285 | 10.5 |
| G | 0.35 | 0.5 | 5.5 | 5.5 | 253 | 5.2 |
| H | 0.35 | 0.5 | 5.5 | 11.0 | 272 | 4.1 |

A comparison between samples D and G shows the positive effect of the wetting agent on the absorption time.

Sample H shows that the pH increase reduces the absorption time considerably without seriously increasing the tearing power.

---

CLAIMS

1. Process in preparing a high absorption fluff pulp from a pulp suspension on a pulp-drying machine, wherein to improve fiberizing, to the pulp suspension before or on the wire is added a debonding agent and optionally, to improve the absorption, an absorption promoting agent, characterized in that the debonding agent is an ampholytic tenside and/or a tenside, which depending on the pH value is cationic or nonionic, said tenside(s) is added in an amount of 0.01-2% by weight based on the dry weight of the pulp suspension, the pH value of the pulp suspension at the addition is kept sufficiently low for the tenside(s) to be in cationic form, one or more absorption promoting agents is added, if required, and the pH value of the pulp after dewatering on the wire is increased to such an extent that the debonding tenside(s) is tranferred into the anionic and nonionic form respectively.

2. Process according to claim 1, characterized in that the absorption promoting agent is at least one anionic tenside.

3. Process according to claim 1, characterized in that the absorption promoting agent is at least one nonionic tenside.

4. Process according to claim 1, characterized in that the absorption promoting agent is at least one fatty alcohol having a straight or branched chain with at least 8 carbon atoms.

5. Process according to any of claims 1-4, characterized in that the pH value of the pulp is increased to transfer the debonding tensides into anionic and nonionic form respectively just before the pulp web leaves the wire.

6. Process according to any of claims 1-4, c h a r a c t e r i z e d in that the pH value of the pulp is increased to transfer the debonding tensides into anionic and nonionic form respectively just before any of the presses.

7. Process according to any of claims 1-4, c h a r a c t e r i z e d in that the pH value of the pulp is increased to transfer the debonding tensides into anionic and nonionic form respectively, when the pulp web is between the last press and the drying section.

8. Process according to any of claims 1-4, c h a r a c t e r i z e d in that the pH value of the pulp is increased to transfer the debonding tensides into anionic and nonionic form respectively in the drying section.

9. Process according to any of claims 1-4, c h a r a c t e r i z e d in that the pH value of the pulp is increased to transfer the debonding tensides into anionic and nonionic form respectively in connection with the feeding of the pulp into the fluffing machine.

European Patent Office

# EUROPEAN SEARCH REPORT

0184603

Application number

EP 84 85 0382

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 617 439 (B.E. CHAPMAN Jr.) * Abstract * | 1 | D 21 C 9/00<br>D 21 D 3/00 |
| A | GB-A-1 407 134 (HONSHU PAPER) * Claims 1-5; pages 4,5 * | 1,3 | |
| A | ABSTRACT BULLETIN OF THE INSTITUTE OF PAPER CHEMISTRY, vol. 54, no. 10, April 1984, page 1221, abstract no. 11489, Appleton, Wisconsin, US; & JP - A - 83 41997 (OJI PAPER CO., LTD.) 11-03-1983 | 1 | |
| A | US-A-4 270 977 (D.F. HERMAN et al.) * Columns 3,12 * | 1,8 | **TECHNICAL FIELDS SEARCHED (Int. Cl.4)** |
| A | US-A-3 989 586 (R.N. BASHAW et al.) * Abstract * | 1-3,8 | D 21 C<br>D 21 D<br>D 21 H |
| A | WO-A-8 200 485 (Y.W. LIM) | | |
| A | US-A-3 844 880 (F.W. MEISEL Jr. et al.) | | |

The present search report has been drawn up for all claims

| Place of search | Date of completion of the search | Examiner |
|---|---|---|
| THE HAGUE | 18-07-1985 | NESTBY K. |

**CATEGORY OF CITED DOCUMENTS**

- X : particularly relevant if taken alone
- Y : particularly relevant if combined with another document of the same category
- A : technological background
- O : non-written disclosure
- P : intermediate document
- T : theory or principle underlying the invention
- E : earlier patent document, but published on, or after the filing date
- D : document cited in the application
- L : document cited for other reasons
- & : member of the same patent family, corresponding document

EPO Form 1503. 03.82